# EUROPEAN PATENT APPLICATION

(11) **EP 4 480 415 A1**
(43) Date of publication of application: **25.12.2024**
(21) Application number: 23756433.1
(22) Date of filing: 16.02.2023
(51) Int. Cl.: A61B 5/291, A61B 5/256, A61B 5/266

(54) **ELETCTRODE FOR BIOSIGNAL MEASUREMENT, BIOSIGNAL MEASUREMENT DEVICE, AND BIOSIGNAL MEASUREMENT METHOD**

(30) Priority: 17.02.2022 JP 2022022658
(71) Applicant: Sumitomo Bakelite Co.Ltd., Shinagawa-ku Tokyo 140-0002 (JP)
(72) Inventor: SAWADA, Masashi, Tokyo 140-0002 (JP); KITAZOE, Katsuma, Tokyo 140-0002 (JP)
(74) Representative: Ter Meer Steinmeister & Partner
(86) International application number: PCT/JP2023/005408
(87) International publication number: WO 2023/157908

(57) **Abstract**

Provided is an electrode for biosignal measurement (electrode (50) for brain wave measurement), which comes into contact with a skin (head portion (99)) of a subject to acquire a biosignal (brain wave signal), the electrode including a protruding portion (60) that protrudes from a base portion (51), an electrode portion (80) that is provided with a conductive member (70) at least in a distal end portion of the protruding portion (60), and a gel-like member (85) that is provided on the protruding portion (60) to cover the electrode portion (80) and has an inside containing moisture.

## Description

### TECHNICAL FIELD

The present invention relates to an electrode for biosignal measurement, a biosignal measurement device, and a biosignal measurement method.

### BACKGROUND ART

Various developments have been made so far in electrodes for biosignal measurement. For example, a technology disclosed in Patent Document 1 is known as this kind of technology.

Patent Document 1 discloses an electrode for brain wave measurement, including a chip portion that comes into contact with a measurement target and receives an electrical signal from the measurement target, a signal transmission unit that receives the electrical signal from the chip portion and transmits the electrical signal to a signal processing device, and a support unit that rotatably supports the chip portion around a rotation axis perpendicular to a contact surface between the chip portion and the measurement target, in which a contact surface between the support unit and the chip portion is coated with conductive grease, projections having conductivity, flexibility, and elasticity are formed on the surface of the chip portion in contact with the measurement target, and the projections are formed of a polymer material or a polymer composition having conductivity and flexibility.

### RELATED DOCUMENT

### PATENT DOCUMENT

[Patent Document 1] Japanese Unexamined Patent Publication No. 2011-120866

### SUMMARY OF THE INVENTION

### TECHNICAL PROBLEM

Meanwhile, in an electrode for biosignal measurement, provided with an electrode portion of a conductive member on projection portions (protruding portions) having a conical shape, biosignal measurement can be appropriately performed by optimizing the shape of the protruding portions, but there is a problem in that contact resistance is large for some subjects and thus brain waves are difficult to acquire.

In the technology disclosed in Patent Document 1, the electrode is configured to have projections integrally extending from columnar chip portions formed of a conductive gel and in a case where the electrode is used by forming the projection portions with a conductive gel and pressing the protruding portions against the skin (here, a head portion) of a subject, there is a problem in terms of the strength and the durability, and therefore, a different technology is required.

The present invention has been made in consideration of the above-described circumstances, and an object of the present invention is to provide a technology that enables stable biosignal measurement by suppressing contact resistance to be low without depending on a subject.

### SOLUTION TO PROBLEM

According to the present invention, the following techniques are provided.
(1) An electrode for biosignal measurement, which comes into contact with skin of a subject to acquire a biosignal, the electrode including: a protruding portion that protrudes from a base portion; an electrode portion that is provided with a conductive member at least in a distal end portion of the protruding portion; and a gel-like member that is provided on the protruding portion to cover the electrode portion and has an inside containing moisture.
(2) The electrode for biosignal measurement according to (1), in which the electrode portion is provided to cover the distal end portion of the protruding portion with the conductive member.
(3) The electrode for biosignal measurement according to (1), in which the protruding portion is provided with a conductive rubber including the conductive member and functions as the electrode portion.
(4) The electrode for biosignal measurement according to any one of (1) to (3), in which the protruding portion has a conical shape.
(5) The electrode for biosignal measurement according to any one of (1) to (4), in which the gel-like member has a spherical shape.
(6) The electrode for biosignal measurement according to any one of (1) to (5), in which the gel-like member is accommodated in a watertight member before use.
(7) The electrode for biosignal measurement according to any one of (1) to (6), in which the gel-like member is exchangeably provided.
(8) The electrode for biosignal measurement according to any one of (1) to (7), in which the biosignal is a brain wave.
(9) A biosignal measurement device including: the electrode for biosignal measurement according to any one of (1) to (8).
(10) A biosignal detection method of measuring a biosignal by mounting the biosignal measurement device according to (9) on a subject.

### ADVANTAGEOUS EFFECTS OF INVENTION

According to the present invention, it is possible to provide a technology that enables stable biosignal measurement by suppressing contact resistance to be low without depending on a subject.

### BRIEF DESCRIPTION OF THE DRAWINGS

Fig. 1 is a view schematically showing a brain wave measurement device according to an embodiment, in a state of being mounted on a human head portion.
Fig. 2 is a perspective view of a frame according to an embodiment.
Fig. 3 is a front view of a brain wave electrode unit according to an embodiment.
Fig. 4 is a perspective view of an electrode for brain wave measurement according to an embodiment.
Fig. 5 is a plan view of the electrode for brain wave measurement according to the embodiment.
Fig. 6 is a cross-sectional view of the electrode for brain wave measurement according to the embodiment, and particularly shows a cross-sectional view taken along the line X1-X1 of Fig. 5.

### DESCRIPTION OF EMBODIMENTS

### <Outline>

Hereinafter, embodiments of the present invention will be described with reference to the accompanying drawings. In the present embodiment, the brain wave will be described as an example of a biosignal. In addition to the brain wave, examples of the biosignal include a cardiac potential, a muscle potential, and a skin potential.

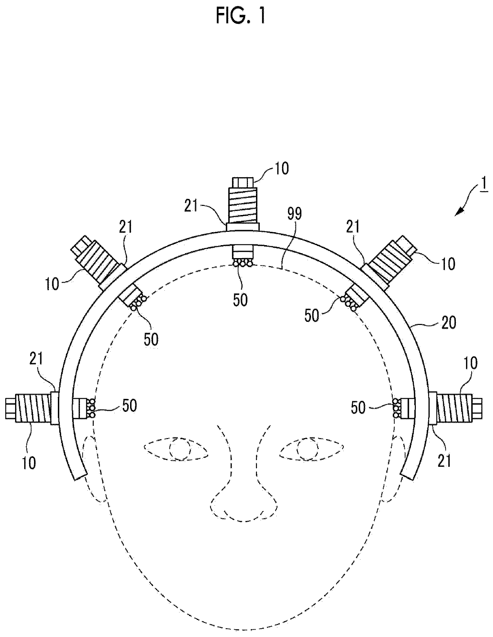

Fig. 1 is a view schematically showing a brain wave measurement device 1 in a state of being mounted on a human head portion 99. A brain wave measurement method of mounting the brain wave measurement device 1 on the head portion 99 of a subject to measure brain waves is performed. The brain wave measurement device 1 is mounted on the head portion 99, detects brain waves as a potential fluctuation from a living body, and outputs the detected brain waves to a brain wave display device (not shown). The brain wave display device acquires the brain wave detected by the brain wave measurement device 1, and performs monitor display, data storage, and known brain wave analysis processing.

### <Structure of Brain Wave Measurement Device 1>

As shown in Fig. 1, the brain wave measurement device 1 has a plurality of brain wave electrode units 10 and a frame 20. In the present embodiment, the brain wave electrode units 10 are provided for five channels (five units).

### <Structure of Frame 20>

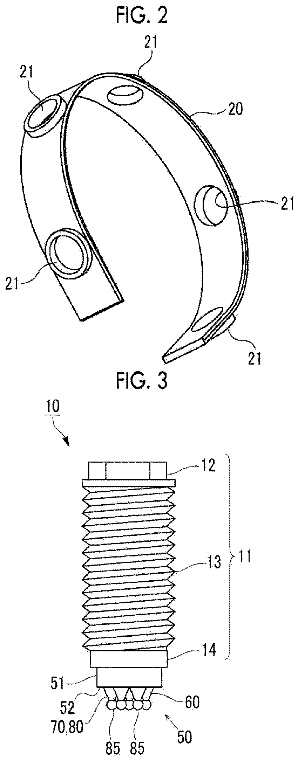

Fig. 2 is a perspective view of the frame 20. The frame 20 is formed of a hard member such as a polyamide resin in a strip shape and is curved to follow the shape of the human head portion 99. Further, in a case where the measurement device is a device that detects a biosignal other than the brain wave, the frame 20 is formed by being deformed to follow the shape of a part (for example, an arm, a chest portion, or an abdominal portion) that acquires a biosignal which is a measurement target.

The frame 20 is provided with five electrode unit attaching units 21 as openings for attaching the brain wave electrode units 10. The positions of the electrode unit attaching units 21 (that is, attachment positions of the brain wave electrode units 10) correspond to positions of T3, C3, Cz, C4, and T4 according to International 10-20 electrode placement method.

The inner peripheral surface of the electrode unit attaching unit 21 is threaded, and the brain wave electrode unit 10 is screwed by a threaded portion 13 of a body portion 11 (see Fig. 3). The amount of protrusion of the brain wave electrode unit 10 to the head portion 99 side is adjusted by adjusting the amount of screwing, and the contact amount and contact pressure with the head portion 99 (scalp) are controlled. In addition, the hair is combed by the operation of screwing the brain wave electrode unit 10.

### <Structure of Brain Wave Electrode Unit 10>

Fig. 3 shows a front view of the brain wave electrode unit 10. The brain wave electrode unit 10 has the substantially columnar body portion 11 and an electrode 50 for brain wave measurement which is provided on one end side (lower side in the drawing) of the body portion 11.

The body portion 11 integrally has a signal extracting unit 12, the threaded portion 13, and an electrode fixing unit 14.

The threaded portion 13 has a shape threaded on a side surface having a cylindrical shape. The signal extracting unit 12 is provided at one end (upper side in the drawing) of the threaded portion 13. A signal output terminal is provided in the signal extracting unit 12, and in a case where the brain wave electrode unit 10 is screwed to the frame 20, the brain wave electrode unit 10 is operated by an operator using a predetermined jig as necessary. The columnar electrode fixing unit 14 is provided at the other end (lower side in the drawing) of the threaded portion 13. The electrode 50 for brain wave measurement is attached to the electrode fixing unit 14.

In the present embodiment, the electrode 50 for brain wave measurement is attached to the frame 20 as a partial configuration of the brain wave electrode unit 10, but the electrode 50 for brain wave measurement may be directly attached to the frame 20.

### <Structure of Electrode 50 for Brain Wave Measurement>

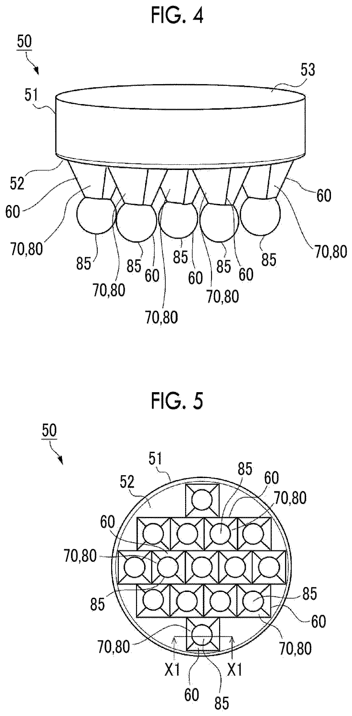

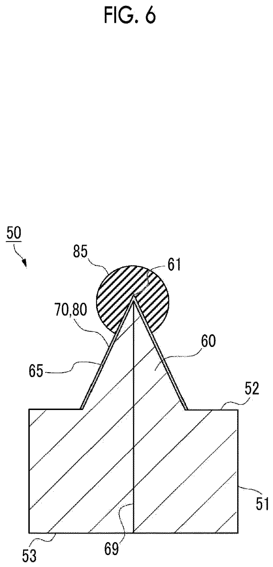

Fig. 4 is a perspective view of the electrode 50 for brain wave measurement. Fig. 5 is a plan view of the electrode 50 for brain wave measurement. Fig. 6 is a cross-sectional view of the electrode 50 for brain wave measurement, and particularly shows a cross-sectional view taken along the line X1-X1 of Fig. 5. Further, the side where a gel-like member 85 is provided is the upper side in Fig. 6 which is different from Fig. 4.

The electrode 50 for brain wave measurement includes a base portion 51, a protruding portion 60, an electrode portion 80, and the gel-like member 85 attached to an electrode portion 80.

The base portion 51 and the protruding portion 60 are integrally provided by a rubber-like elastic body. A specific material of the elastic body will be described later. Further, the base portion 51 and the protruding portion 60 are not limited to the configuration in which the base portion 51 and the protruding portion 60 are integrally provided, and may have a configuration in which the base portion 51 and the protruding portion 60, which are separately provided, are assembled using an adhesive or a fitting structure. The entirety of the electrode 50 for brain wave measurement is not necessarily a rubber-like elastic body, and the protruding portion 60 may be formed by a rubber-like elastic body.

The base portion 51 has a substantially columnar shape, and one end thereof is a protruding portion forming surface 52 having a circular shape and the other end thereof is an attachment surface 53 having a circular shape. The attachment surface 53 is attached to the electrode fixing unit 14 of the body portion 11 by an adhesive or the like. A fixing structure of the attachment surface 53 and the electrode fixing unit 14 is not particularly limited, and for example, a fitting structure by an uneven shape may be used.

### <Structure of Protruding Portion 60>

A plurality of the protruding portions 60 having a pyramidal shape (polygonal pyramidal shape) are provided in alignment on the protruding portion forming surface 52. More specifically, the protruding portion 60 has a square bottom surface and is a quadrangular pyramid, which is a so-called "pyramid type", formed such that the perpendicular line from the apex (distal end portion 61) to the bottom surface (that is, the protruding portion forming surface 52) passes through the centroid of the bottom surface.

The fifteen protruding portions 60 according to the present embodiment are arranged in five rows on the protruding portion forming surface 52 in the top view shown in Fig. 5. More specifically, the protruding portions 60 are arranged such that one protruding portion 60 provided in the first line, four protruding portions 60 provided in the second line, five protruding portions 60 provided in the third line, four protruding portions 60 provided in the fourth line, and one protruding portion 60 provided in the fifth line are symmetrical in the vertical direction and the horizontal direction as described above.

The width of the protruding portion 60 (that is, the width of the bottom surface of the apex of the polygonal pyramidal shape) is appropriately set according to the strength in a case of pressing the protruding portion against the head portion 99, the number of the protruding portions 60, and the size and the strength of the gel-like member 85 to be attached. For example, the width of the protruding portion 60 can be set to 2 mm or greater and 10 mm or less.

The height of the protruding portion 60 is appropriately set according to the strength in a case of pressing the protruding portion against the head portion 99, the number of the protruding portions 60, and the size and the strength of the gel-like member 85 to be attached. For example, the height of the protruding portion 60 can be set to 2 mm or greater and 20 mm or less.

### <Structure of Conductive Member 70 (Electrode Portion 80)>

As shown in the cross-sectional view of the protruding portion 60 in Fig. 6, a conductive member 70 is formed to cover a protruding portion inclined surface 62 of the protruding portion 60. In the present embodiment, the conductive member 70 is provided to cover the distal end portion 61 (apex of the conical body) of the protruding portion 60. Further, the conductive member 70 may be formed in a region covered with the gel-like member 85 attached to the protruding portion 60. That is, the distal end portion 61 is not necessarily covered with the conductive member 70 in a case where the gel-like member 85 and the electrode portion 80 can be electrically conductive. In a case where the gel-like member 85 is configured to be removable and use of the gel-like member 85 without attaching the gel-like member 85 is assumed, it is preferable that the distal end portion 61 is covered with the conductive member 70.

### <Gel-like Member 85>

The gel-like member 85 is a member obtained by forming, in a spherical shape, the gel-like material (also referred to as a hydrogel) containing moisture inside and is attached to be pierced into the distal end portion 61 of the protruding portion 60.

In a case where the electrode 50 for brain wave measurement is pressed against the head portion 99 for the brain wave measurement, the gel-like member 85 comes into contact with the head portion 99. At this time, the electrolytic substance (generally, salt) of the scalp is taken into the gel-like member 85. As a result, the electrode portion 80 and the scalp are in an electrically conductive state.

The size of the gel-like member 85 may be the size set such that the adjacent gel-like member 85 does not come into contact with the protruding portion 60 in a case where the gel-like member 85 is attached to the protruding portion 60, and the gel-like member can be set to have a diameter of, for example, 5 mm to 20 mm. Further, the gel-like member 85 may have a shape of an ellipsoid (prolate spheroid) or a shape in which a part of the shape is cut out instead of a perfect sphere, or may further have a three-dimensional shape such as a tetrahedron. Further, from the viewpoint of dispersing the force in a case where the gel-like member 85 comes into contact with the head portion 99, a shape in which the portion in contact with the head portion 99 is a curved surface is preferable.

The gel-like material is not particularly limited as long as the material can contain a sufficient amount of water and achieve sufficient strength and flexibility in a case where the gel-like member 85 is pressed against the head portion 99, and for example, an acrylic hydrogel or a silicone-based hydrogel can be used. In a case where the acrylic hydrogel is a salt water-free gel, the moisture content can be set to be in a range of 15% to 25% by weight, and in a case where the acrylic hydrogel is a salt water-free gel, the salt water content can be set to be in a range of 20% to 30% by weight, and the salt content can be set to be in a range of 4% to 6% by weight.

The gel-like member 85 may be removable or exchangeable. In a case where the gel-like member is removable, the gel-like member 85 can be used by a method of accommodating the gel-like member in a container consisting of a watertight member until immediately before use by the brain wave measurement, attaching the gel-like member 85 to the protruding portion 60 during use, and accommodating the gel-like member 85 in the container after use. In a case where the gel-like member 85 is exchangeable, a plurality of kinds of gel-like members 85 having different sizes, shapes, and materials may be prepared, and an appropriate gel-like member 85 may be selectable according to the state of the scalp of the subject, the use environment, and the like.

### <Structure of signal line 69>

As shown in the cross-sectional shape of the electrode 50 for brain wave measurement in Fig. 6, a conductive signal line 69 connected to the electrode portion 80 is provided inside the protruding portion 60. As the signal line 69, various arrangement structures can be employed in a case of an aspect in which the inside of the protruding portion 60 is electrically conductive. For example, a distal end of the signal line 69 may have a structure which protrudes with respect to the distal end of the protruding portion 60 or the inclined surface of the distal end portion of the protruding portion 60, a structure which is substantially on the same plane, or a structure which is buried. From the viewpoint of connection stability with the electrode portion 80, the protruding structure may be used. A part or the entirety of the protruding portion of the distal end of the signal line 69 is covered with the electrode portion 80.

As the protruding structure of the distal end of the signal line 69, a structure without folding, a structure with folding, or a structure in which the signal line 69 is wound around the surface of the distal end portion of the protruding portion 60 may be employed. In addition, the signal line 69 may be inclined with respect to the perpendicular line without coinciding with the perpendicular line extending from the apex (the distal end portion 61) of the protruding portion 60.

### <Material of Electrode 50 for Brain Wave Measurement (Base Portion 51 or Protruding Portion 60)>

The material of the electrode 50 for brain wave measurement (the base portion 51 or the protruding portion 60) will be described. The electrode 50 for brain wave measurement is configured to have a rubber-like elastic body. Specifically, the rubber-like elastic body is rubber or a thermoplastic elastomer (also simply referred to as "elastomer (TPE)"). Examples of the rubber include silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), amide-based TPE (TPAE), and the like.

In a case where the electrode 50 for brain wave measurement is formed of silicone rubber, in a case where a type A durometer hardness of the surface of the electrode 50 for brain wave measurement (the protruding portion 60 or the base portion 51), which is measured at 37°C in conformity with JIS K 6253 (1997), is defined as a rubber hardness A, the rubber hardness A is, for example, 15 or greater and 55 or less.

Here, the above-described silicone rubber-based curable composition will be described.

The above-described silicone rubber may be configured with a cured substance of the silicone rubber-based curable composition. A curing step of the silicone rubber-based curable resin composition is performed by heating (primary curing) the composition, for example, at 100°C to 250°C for 1 to 30 minutes and post-baking (secondary curing) the heated composition at 100°C to 200°C for 1 to 4 hours.

The silicone rubber may be any of insulating silicone rubber or conductive silicone rubber. Insulating silicone rubber is silicone rubber not containing a conductive filler, and conductive silicone rubber is silicone rubber containing a conductive filler.

The silicone rubber-based curable composition according to the present embodiment may contain a vinyl group-containing organopolysiloxane (A). The vinyl group-containing organopolysiloxane (A) is a polymer containing the silicone rubber-based curable composition according to the present embodiment as a main element.

The insulating silicone rubber-based curable composition and the conductive silicone rubber-based curable composition may contain the same kind of vinyl group-containing linear organopolysiloxane. The same type of vinyl group-containing linear organopolysiloxanes may contain at least vinyl groups having the same functional groups and may be linear. The amount of vinyl groups in a molecule, the molecular weight distribution, or the addition amounts thereof may vary between the vinyl group-containing linear organopolysiloxanes.

The insulating silicone rubber-based curable composition and the conductive silicone rubber-based curable composition may further contain different kinds of vinyl group-containing organopolysiloxanes.

The vinyl group-containing organopolysiloxane (A) can contain a vinyl group-containing linear organopolysiloxane (A1) having a linear structure.

The vinyl group-containing linear organopolysiloxane (A1) has a linear structure and contains a vinyl group which functions as a crosslinking point during curing.

The content of the vinyl group in the vinyl group-containing linear organopolysiloxane (A1) is not particularly limited. For example, the vinyl group in the vinyl group-containing linear organopolysiloxane (A1) has 2 or more vinyl groups in a molecule, and the content of the vinyl groups is preferably 15% by mole or less and more preferably 0.01% to 12% by mole. In a case where the content of the vinyl group is in the above range, the amount of the vinyl group in the vinyl group-containing linear organopolysiloxane (A1) can be optimized, and a network between the respective components, which will be described later, can be reliably formed. In the present embodiment, a range represented by "to" includes numerical values of both ends.

In the present specification, the vinyl group content is in units of % by mole of a vinyl group-containing siloxane unit with respect to 100% by mole of all the units forming the vinyl group-containing linear organopolysiloxane (A1). Here, it is assumed that there may be one vinyl group for each vinyl group-containing siloxane unit.

The polymerization degree of the vinyl group-containing linear organopolysiloxane (A1) is not particularly limited and is, for example, preferably in a range of about 1,000 to 10,000 and more preferably in a range of about 2,000 to 5,000. The polymerization degree can be determined, for example, as a polystyrene-equivalent number-average polymerization degree (or number-average molecular weight) or the like gel permeation chromatography (GPC) using chloroform as an elution solvent.

The specific gravity of the vinyl group-containing linear organopolysiloxane (A1) is not particularly limited and is preferably in a range of about 0.9 to 1.1.

Using the vinyl group-containing linear organopolysiloxane (A1) having the polymerization degree and the specific gravity in the ranges described above makes it possible to obtain silicone rubber having higher heat resistance, higher flame retardancy, higher chemical stability, and the like.

As the vinyl group-containing linear organopolysiloxane (A1), a structure represented by Formula (1) is particularly preferable.

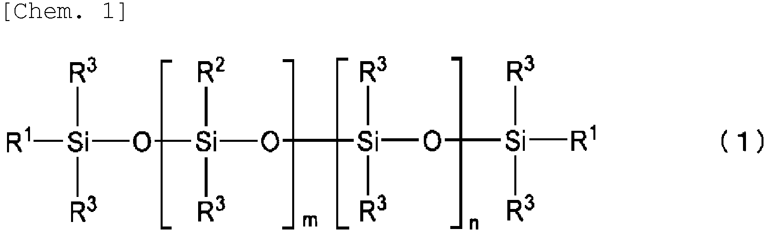

In Formula (1), R¹ represents a substituted or unsubstituted alkyl group, alkenyl group, or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Among these, a vinyl group is preferable. Examples of the aryl group having 1 to 10 carbon atoms include a phenyl group and the like.

R² represents a substituted or unsubstituted alkyl group, alkenyl group, or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

R³ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group.

Examples of substituents of R¹ and R² in Formula (1) include a methyl group, a vinyl group, and the like. Examples of substituents of R³ include a methyl group and the like.

In Formula (1), a plurality of R¹'s is independent of each other, and may be the same as or different from each other. The same shall be applied to R² and R³.

m and n each represent the number of repeating units configuring the vinyl group-containing linear organopolysiloxane (A1) represented by Formula (1). m represents an integer of 0 to 2,000, and n represents an integer of 1,000 to 10,000. m is preferably 0 to 1,000, and n is preferably 2,000 to 5,000.

Examples of specific structures of the vinyl group-containing linear organopolysiloxane (A1) represented by Formula (1) include a structure represented by Formula (1-1).

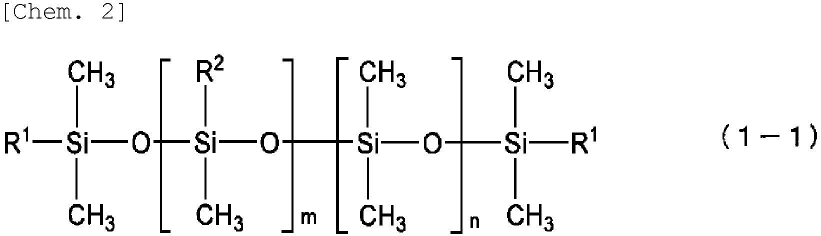

In Formula (1-1), R¹ and R² each independently represent a methyl group or a vinyl group, and at least one of R¹ or R² represents a vinyl group.

It is preferable that the vinyl group-containing linear organopolysiloxane (A1) contains a first vinyl group-containing linear organopolysiloxane (A1-1) that has two or more vinyl groups in a molecule and has a vinyl group content of 0.4% by mole or less and a second vinyl group-containing linear organopolysiloxane (A1-2) that has a vinyl group content of 0.5% to 15% by mole. Combining the first vinyl group-containing linear organopolysiloxane (A1-1) having the general vinyl group content and the second vinyl group-containing linear organopolysiloxane (A1-2) having a high vinyl group content as raw rubber that is a raw material of the silicone rubber enables vinyl groups to be unevenly distributed, which makes it possible to more effectively form variations in the crosslinking density in the crosslinked network of the silicone rubber. As a result, the tear strength of the silicone rubber can be more effectively improved.

Specifically, as the vinyl group-containing linear organopolysiloxane (A1), for example, it is preferable to use the first vinyl group-containing linear organopolysiloxane (A1-1) in which, in Formula (1-1), two or more of an unit in which R¹ represents a vinyl group and/or an unit in which R² represents a vinyl group are included in a molecule and the content of the units is 0.4% by mole or less, and the second vinyl group-containing linear organopolysiloxane (A1-2) in which the unit in which R¹ represents a vinyl group and/or the unit in which R² represents a vinyl group are included and the content of the units is 0.5% to 15% by mole.

The first vinyl group-containing linear organopolysiloxane (A1-1) preferably has a vinyl group content of 0.01% to 0.2% by mole. In addition, the second vinyl group-containing linear organopolysiloxane (A1-2) preferably has a vinyl group content of 0.8% to 12% by mole.

In a case where the first vinyl group-containing linear organopolysiloxane (A1-1) and the second vinyl group-containing linear organopolysiloxane (A1-2) are mixed together, the ratio between (A1-1) and (A1-2) is not particularly limited. For example, the weight ratio (A1-1):(A1-2) is preferably 50:50 to 95:5, and more preferably 80:20 to 90:10.

As each of the first and second vinyl group-containing linear organopolysiloxanes (A1-1) and (A1-2), only one compound may be used, or two or more compounds may be used in combination.

The vinyl group-containing organopolysiloxane (A) may contain a vinyl group-containing branched organopolysiloxane (A2) having a branched structure.

### <<Organohydrogen polysiloxane (B)>>

The silicone rubber-based curable composition of the present embodiment may contain a crosslinking agent. The crosslinking agent can contain an organohydrogen polysiloxane (B) .

The organohydrogen polysiloxane (B) is classified into a linear organohydrogen polysiloxane (B1) having a linear structure and a branched organohydrogen polysiloxane (B2) having a branched structure, and can include either or both of these.

The insulating silicone rubber-based curable composition and the conductive silicone rubber-based curable composition may contain the same type of crosslinking agents. The same type of crosslinking agents may have at least a common structure such as a linear structure or a branched structure. The crosslinking agents may have different molecular weight distributions or different functional groups in a molecule, and the addition amounts thereof may be different.

The insulating silicone rubber-based curable composition and the conductive silicone rubber-based curable composition may further contain different types of crosslinking agents.

The linear organohydrogen polysiloxane (B1) is a polymer that has a linear structure and a structure (=Si-H) in which hydrogen is directly bonded to Si, and has a hydrosilylation reaction with vinyl groups contained in the components mixed with the silicone rubber-based curable composition in addition to the vinyl groups of the vinyl group-containing organopolysiloxane (A) to crosslink the components.

The molecular weight of the linear organohydrogen polysiloxane (B1) is not particularly limited. For example, the weight-average molecular weight thereof is preferably less than or equal to 20,000 and more preferably greater than or equal to 1,000 and less than or equal to 10,000.

The weight-average molecular weight of the linear organohydrogen polysiloxane (B1) can be measured as a polystyrene-equivalent value by gel permeation chromatography (GPC) using chloroform as an elution solvent.

It is preferable that the linear organohydrogen polysiloxane (B1) contains no vinyl group in general. In a case where the linear organohydrogen polysiloxane (B1) does not have a vinyl group, it is possible to reliably prevent the occurrence of a crosslinking reaction in a molecule of the linear organohydrogen polysiloxane (B1).

As the linear organohydrogen polysiloxane (B1), for example, a compound having a structure represented by Formula (2) is preferably used.

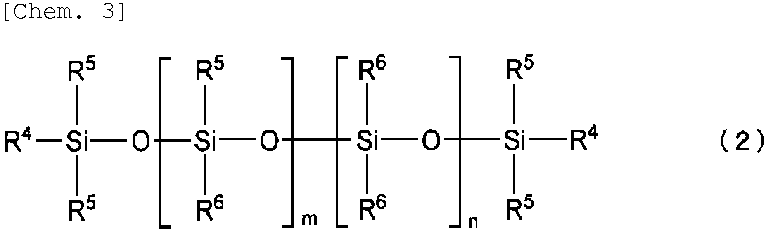

In Formula (2), R⁴ represents a substituted or unsubstituted alkyl group, alkenyl group, or aryl group having 1 to 10 carbon atoms, a hydrocarbon group as a combination of these, or a hydride group. Examples of the alkyl group having 1 to 10 carbon atoms include a methyl group, an ethyl group, a propyl group, and the like. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

R⁵ represents a substituted or unsubstituted alkyl group, alkenyl group, or aryl group having 1 to 10 carbon atoms, a hydrocarbon group as a combination of these, or a hydride group. Examples of the alkyl group having 1 to 10 carbon atoms include a methyl group, an ethyl group, and a propyl group. Among these, a methyl group is preferable. Examples of the alkenyl group having 1 to 10 carbon atoms include a vinyl group, an allyl group, a butenyl group, and the like. Examples of the aryl group having 1 to 10 carbon atoms include a phenyl group.

In Formula (2), a plurality of R⁴'s is independent of each other, and may be the same as or different from each other. The same shall be applied to R⁵. Here, at least two or more among the plurality of R⁴'s and the plurality of R⁵'s represent hydride groups.

R⁶ represents a substituted or unsubstituted alkyl group or aryl group having 1 to 8 carbon atoms, or a hydrocarbon group as a combination of these. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. A plurality of R⁶'s is independent of each other, and may be the same as or different from each other.

Examples of substituents of R⁴, R⁵, and R⁶ in Formula (2) include a methyl group, a vinyl group, and the like. From the viewpoint of preventing an intramolecular crosslinking reaction, a methyl group is preferable.

m and n each independently represent the number of repeating units configuring the linear organohydrogen polysiloxane (B1) represented by Formula (2), m represents an integer of 2 to 150, and n represents an integer of 2 to 150. It is preferable that m represents an integer of 2 to 100 and n represents an integer of 2 to 100.

As the linear organohydrogen polysiloxane (B1), only one compound may be used alone, or two or more compounds may be used in combination.

Having a branched structure, the branched organohydrogen polysiloxane (B2) is a component that largely contributes to the formation of a density-varying structure of the crosslinking density in the silicone rubber system by forming a region having a high crosslinking density. In addition, similarly to the linear organohydrogen polysiloxane (B1), the branched organohydrogen polysiloxane (B2) is a polymer that has a structure (=Si-H) in which hydrogen is directly bonded to Si, and has a hydrosilylation reaction with vinyl groups of components mixed with the silicone rubber-based curable composition in addition to the vinyl groups of the vinyl group-containing organopolysiloxane (A) to crosslink these components.

The specific gravity of the branched organohydrogen polysiloxane (B2) is in a range of 0.9 to 0.95.

It is preferable that the branched organohydrogen polysiloxane (B2) contains no vinyl group in general. In a case where the branched organohydrogen polysiloxane (B2) does not have a vinyl group, it is possible to reliably prevent the occurrence of a crosslinking reaction in a molecule of the branched organohydrogen polysiloxane (B2).

The branched organohydrogen polysiloxane (B2) is preferably represented by the following Average Compositional Formula (c).

Average Compositional Formula (c) (Hₐ(R⁷)₃₋ₐSiO_{1/2})ₘ(SiO_{4/2})ₙ

(In Formula (c), R⁷ represents a monovalent organic group, a represents an integer of 1 to 3, m represents the number of Hₐ(R⁷)₃₋ₐSiO_{1/2} units, and n represents the number of SiO_{4/2} units.)

In Formula (c), R⁷ represents a monovalent organic group, and preferably represents a substituted or unsubstituted alkyl group or aryl group having 1 to 10 carbon atoms, or a hydrocarbon group as a combination of these. Examples of the alkyl group having 1 to 10 carbon atoms include a methyl group, an ethyl group, a propyl group, and the like. Among these, a methyl group is preferable. Examples of the aryl group having 1 to 10 carbon atoms include a phenyl group.

In Formula (c), a represents the number of hydride groups (hydrogen atoms directly bonded to Si) which is an integer of 1 to 3. a is preferably 1.

In addition, in Formula (c), m represents the number of Hₐ(R⁷)₃₋ₐSiO_{1/2} units, and n represents the number of SiO_{4/2} units.

The branched organohydrogen polysiloxane (B2) has a branched structure. The difference between the linear organohydrogen polysiloxane (B1) and the branched organohydrogen polysiloxane (B2) is whether the structure thereof is linear or branched. Assuming that the number of Si is 1, the number of alkyl groups R bonded to Si is in a range of 1.8 to 2.1 in the linear organohydrogen polysiloxane (B1) and is in a range of 0.8 to 1.7 in the branched organohydrogen polysiloxane (B2).

The residual amount of the branched organohydrogen polysiloxane (B2) is greater than or equal to 5% due to having a branched structure in a case of being heated, for example, to 1,000°C at a heating rate of 10 °C/min in a nitrogen atmosphere. On the other hand, having a linear structure, the linear organohydrogen polysiloxane (B1) substantially does not leave residues in a case where the compound is heated under the above conditions.

Specific examples of the branched organohydrogen polysiloxane (B2) include compounds having a structure represented by Formula (3).

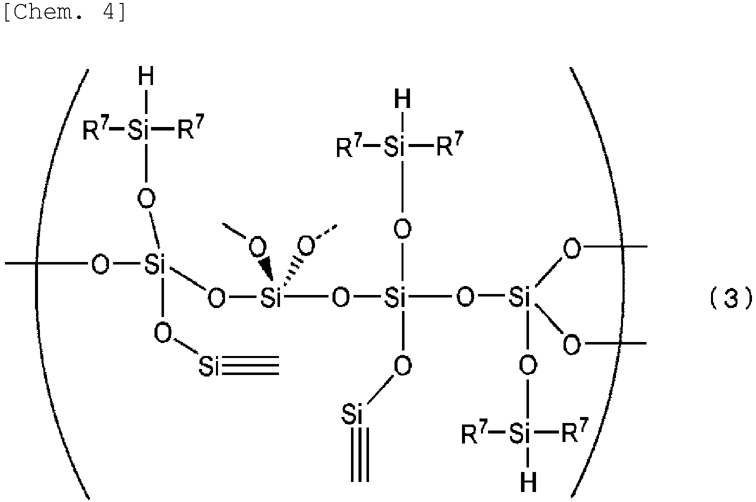

In Formula (3), R⁷ represents a substituted or unsubstituted alkyl group or aryl group having 1 to 8 carbon atoms, a hydrocarbon group as a combination of these, or a hydrogen atom. Examples of the alkyl group having 1 to 8 carbon atoms include a methyl group, an ethyl group, a propyl group, and the like. Among these, a methyl group is preferable. Examples of the aryl group having 1 to 8 carbon atoms include a phenyl group. Examples of substituents of R⁷ include a methyl group and the like.

In Formula (3), a plurality of R⁷'s is independent of each other, and may be the same as or different from each other.

In Formula (3), "-O-Si=" means that Si has a branched structure that expands three-dimensionally.

As the branched organohydrogen polysiloxane (B2), only one compound may be used alone, or two or more compounds may be used in combination.

In each of the linear organohydrogen polysiloxane (B1) and the branched organohydrogen polysiloxane (B2), the amount of hydrogen atoms (hydride groups) directly bonded to Si is not particularly limited. Here, in the silicone rubber-based curable composition, the total amount of hydride groups in the linear organohydrogen polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). In a case where the total amount of hydride groups is in the above range, a crosslinked network can be reliably formed among the linear organohydrogen polysiloxane (B1) and the branched organohydrogen polysiloxane (B2), and the vinyl group-containing linear organopolysiloxane (A1).

### <<Silica particles (C)>>

The silicone rubber-based curable composition of the present embodiment contains a non-conductive filler. As necessary, the non-conductive filler may contain silica particles (C). In a case where the silicone rubber-based curable composition contains the silica particles (C), the hardness or mechanical strength of the elastomer can be improved.

The insulating silicone rubber-based curable composition and the conductive silicone rubber-based curable composition may contain the same type of non-conductive fillers. The same type of non-conductive fillers may have at least a common constituent material, and the particle diameter, specific surface area, surface treatment agent, or the addition amount thereof may vary between the fillers.

The insulating silicone rubber-based curable composition and the conductive silicone rubber-based curable composition may further contain different types of silane coupling agents.

The silica particles (C) are not particularly limited. For example, fumed silica, sintered silica, precipitated silica, or the like is used. These may be used alone or in combination of two or more kinds thereof.

The specific surface area of the silica particles (C) measured using, for example, a BET method is, for example, preferably 50 to 400 m²/g, and more preferably 100 to 400 m²/g. The average primary particle diameter of the silica particles (C) is, for example, preferably 1 to 100 nm and more preferably about 5 to 20 nm.

Using the silica particles (C) having the specific surface area and the average particle diameter in the above ranges makes it possible to improve the hardness and the mechanical strength of the formed silicone rubber and, particularly, to improve the tensile strength of the formed silicone rubber.

### <<Silane coupling agent (D)>>

The silicone rubber-based curable composition according to the present embodiment may contain a silane coupling agent (D).

The silane coupling agent (D) may have a hydrolyzable group. The hydrolyzable group is hydrolyzed into a hydroxyl group by water. The hydroxyl group has a dehydration condensation reaction with a hydroxyl group on the surface of the silica particles (C), which can modify the surface of the silica particles (C).

The insulating silicone rubber-based curable composition and the conductive silicone rubber-based curable composition may contain the same kind of silane coupling agent. The same type of silane coupling agents may have at least a common functional group and may have other functional groups in a molecule, and the addition amounts thereof may be different.

The insulating silicone rubber-based curable composition and the conductive silicone rubber-based curable composition may further contain different types of silane coupling agents.

The silane coupling agent (D) can include a silane coupling agent having a hydrophobic group. In this case, the hydrophobic group is added to the surfaces of the silica particles (C). Accordingly, in the silicone rubber-based curable composition and the silicone rubber, the cohesive force between the silica particles (C) decreases (cohesion through a hydrogen bond formed by a silanol group weakens). Presumably, as a result, the dispersibility of the silica particles (C) in the silicone rubber-based curable composition may be improved. Consequently, the interface between the silica particles (C) and a rubber matrix enlarges, and a reinforcing effect of the silica particles (C) increases. Furthermore, presumably, the silica particles (C) in the matrix may become more slippery during the matrix deformation of rubber. By improving the dispersibility and slipperiness of the silica particles (C), a mechanical strength (for example, a tensile strength, a tear strength, or the like) of the silicone rubber by the silica particles (C) is improved.

The silane coupling agent (D) may include a silane coupling agent having a vinyl group. In a case where the silane coupling agent has a vinyl group, the vinyl group is introduced into the surface of the silica particles (C). Therefore, in a case where the silicone rubber-based curable composition is cured, that is, in a case where the vinyl groups of the vinyl group-containing organopolysiloxane (A) and the hydride groups in the organohydrogen polysiloxane (B) have a hydrosilylation reaction to form a network (crosslinked structure) using this reaction, the vinyl groups of the silica particles (C) are also involved in the hydrosilylation reaction with the hydride groups of the organohydrogen polysiloxane (B). As a result, the silica particles (C) are also incorporated into the network. Accordingly, it is possible to achieve the reduction in hardness and the increase in modulus of the formed silicone rubber.

As the silane coupling agent (D), a silane coupling agent having a hydrophobic group and a silane coupling agent having a vinyl group can be used in combination.

Examples of the silane coupling agent (D) include a silane coupling agent represented by Formula (4).

Yₙ-Si-(X) ₄₋ₙ (4)

In Formula (4), n represents an integer of 1 to 3. Y represents any functional group of a hydrophobic group, a hydrophilic group, or a vinyl group, in a case where n represents 1, Y represents a hydrophobic group, and in a case where n represents 2 or 3, at least one of Y's represents a hydrophobic group. X represents a hydrolyzable group.

The hydrophobic group is an alkyl group or aryl group having 1 to 6 carbon atoms, or a hydrocarbon group as a combination of these. Examples of the hydrophobic group include a methyl group, an ethyl group, a propyl group, a phenyl group, and the like. Among these, a methyl group is particularly preferable.

Examples of the hydrophilic group include a hydroxyl group, a sulfonate group, a carboxyl group, a carbonyl group, and the like. Among these, a hydroxyl group is particularly preferable. The hydrophilic group may be incorporated into the silane coupling agent as a functional group. From the viewpoint of making the silane coupling agent (D) hydrophobic, it is preferable that the silane coupling agent (D) contains no hydrophilic group.

Examples of the hydrolyzable group include an alkoxy group such as a methoxy group or an ethoxy group, a chloro group, a silazane group, and the like. Among these, in view of high reactivity with the silica particles (C), a silazane group is preferable. Owing to its structural characteristics, the silane coupling agent having a silazane group as a hydrolyzable group has two structures represented by (Yₙ-Si-) in Formula (4).

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, or decyltrimethoxysilane; a chlorosilane such as methyltrichlorosilane, dimethyldichlorosilane, trimethylchlorosilane, or phenyltrichlorosilane; and hexamethyldisilazane. Among these, a silane coupling agent having a trimethylsilyl group that includes one or more compounds selected from the group consisting of hexamethyldisilazane, trimethylchlorosilane, trimethylmethoxysilane, and trimethylethoxysilane is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, or vinylmethyldimethoxysilane; a chlorosilane such as vinyltrichlorosilane or vinylmethyldichlorosilane; and divinyltetramethyldisilazane. Among these, a silane coupling agent having a vinyl group-containing organosilyl group that includes one or more compounds selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane is preferable.

In addition, in a case where the silane coupling agent (D) includes two kinds including the silane coupling agent having a trimethylsilyl group and the silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited, but a weight ratio (D1):(D2) is 1:0.001 to 1:0.35, preferably 1:0.01 to 1:0.20 and more preferably 1:0.03 to 1:0.15. In a case where the ratio is set to be in the above-described numerical ranges, desired physical properties of silicone rubber can be obtained. Specifically, a balance between the dispersibility of silica in the rubber and the crosslinkability of the rubber can be achieved.

In the present embodiment, the lower limit of the content of the silane coupling agent (D) with respect to the total amount of 100 parts by weight of the vinyl group-containing organopolysiloxane (A) is preferably greater than or equal to 1% by mass, more preferably greater than or equal to 3% by mass, and still more preferably greater than or equal to 5% by mass. Furthermore, the upper limit of the content of the silane coupling agent (D) with respect to the total amount of 100 parts by weight of the vinyl group-containing organopolysiloxane (A) is preferably less than or equal to 100% by mass, more preferably less than or equal to 80% by mass, and still more preferably less than or equal to 40% by mass.

Setting the content of the silane coupling agent (D) to be equal to or more than the aforementioned lower limit makes it possible to improve the adhesion between a columnar portion containing an elastomer and the conductive resin layer. In addition, the mechanical strength of the silicone rubber can be improved. Furthermore, setting the content of the silane coupling agent (D) to be less than or equal to the aforementioned upper limit makes it possible to obtain silicone rubber having appropriate mechanical characteristics.

### <<Platinum or platinum compound (E)>>

The silicone rubber-based curable composition according to the present embodiment may contain a catalyst. The catalyst can include platinum or platinum compound (E). The platinum or platinum compound (E) is a catalyst component that functions as a catalyst during curing. The addition amount of the platinum or platinum compound (E) is the amount of the catalyst.

The insulating silicone rubber-based curable composition and the conductive silicone rubber-based curable composition may contain the same type of catalysts. The same type of catalysts may have at least a common constituent material. Different compositions may be contained in the catalysts, or the addition amounts thereof may vary between the catalysts.

The insulating silicone rubber-based curable composition and the conductive silicone rubber-based curable composition may further contain different catalysts.

As the platinum or platinum compound (E), known platinum or platinum compounds can be used. Examples thereof include platinum black, silica or carbon black on which platinum is supported, platinic chloride or an alcohol solution of platinic chloride, a complex salt of platinic chloride and olefin, a complex salt of platinic chloride and vinylsilxoane, and the like.

As the platinum or platinum compound (E), only one platinum element or one platinum compound may be used alone, or two or more platinum elements or two platinum compounds may be used in combination.

In the present embodiment, the content of the platinum or platinum compound (E) in the silicone rubber-based curable composition refers to the amount of the catalyst and may be appropriately set. Specifically, the content of platinum group metals by weight with respect to the total amount of 100 parts by weight of the vinyl group-containing organopolysiloxane (A), the silica particles (C), and the silane coupling agent (D) is 0.01 to 1000 ppm and preferably 0.1 to 500 ppm.

Setting the content of the platinum or platinum compound (E) to be equal to or more than the aforementioned lower limit makes it possible to cure the silicone rubber-based curable composition at an appropriate rate. In addition, setting the content of the platinum or platinum compound (E) to be equal to or less than the aforementioned upper limit makes it possible to reduce manufacturing costs.

### <<Water (F)>>

In addition, the silicone rubber-based curable composition according to the present embodiment may contain water (F) in addition to the above-described components (A) to (E).

The water (F) is a component that functions as a dispersion medium for dispersing the respective components in the silicone rubber-based curable composition and contributes to the reaction between the silica particles (C) and the silane coupling agent (D). Therefore, the silica particles (C) and the silane coupling agent (D) can be more reliably linked to each other in the silicone rubber, and the silicone rubber can show uniform characteristics as a whole.

### (Other components)

Further, the silicone rubber-based curable composition according to the present embodiment may further contain other components in addition to the above-described components (A) to (F). Examples of those other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, or mica, and an additive such as a reaction inhibitor, a dispersant, a pigment, a dye, an antistatic agent, an antioxidant, a flame retardant, or a thermal conductivity enhancing agent.

The conductive solution (conductive silicone rubber composition) according to the present embodiment contains the above-described conductive filler and a solvent, in addition to the above-described silicone rubber-based curable composition not containing the conductive filler.

As the above-described solvent, various well-known solvents can be used, and for example, a high-boiling point solvent can be contained. These may be used alone or in combination of two or more kinds thereof.

Examples of the solvent include aliphatic hydrocarbons such as pentane, hexane, cyclohexane, heptane, methylcyclohexane, ethylcyclohexane, octane, decane, dodecane, and tetradecane; aromatic hydrocarbons such as benzene, toluene, ethylbenzene, xylene, trifluoromethylbenzene, and benzotrifluoride; ethers such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; haloalkanes such as dichloromethane, chloroform, 1,1-dichloroethane, 1,2-dichloroethane, 1,1,1-trichloroethane, and 1,1,2-trichloroethane; carboxylic acid amides such as N,N-dimethylformamide and N,N-dimethylacetamide; sulfoxides such as dimethyl sulfoxide and diethyl sulfoxide, and the like. These may be used alone or in combination of two or more kinds thereof.

By adjusting the solid content in the solution, the above-described conductive solution can have an appropriate viscosity for various coating methods such as spray coating and dip coating.

Further, in a case where the conductive solution contains the conductive filler and the silica particles (C), the lower limit of the content of the silica particles (C) contained in the electrode 50 for brain wave measurement is, for example, 1% by mass or greater, preferably 3% by mass or greater, and more preferably 5% by mass or greater with respect to 100% by mass of the total amount of the silica particles (C) and the conductive filler. In this manner, the mechanical strength of the electrode 50 for brain wave measurement can be improved. Meanwhile, the upper limit of the content of the silica particles (C) contained in the electrode 50 for brain wave measurement is, for example, 20% by mass or less, preferably 15% by mass or less, and more preferably 10% by mass or less with respect to 100% by mass of the total amount of the silica particles (C) and the conductive filler. In this manner, the balance between the conductivity, the mechanical strength, and the flexibility of the electrode 50 for brain wave measurement can be achieved.

By optionally heating and drying the conductive solution, the conductive silicone rubber is obtained.

The conductive silicone rubber may be configured such that it does not contain silicone oil. In this manner, a decrease in conductivity due to the bleeding out of the silicone oil on the surface of the electrode 50 for brain wave measurement (protruding portion 60) can be suppressed.

### <Material of signal line 69>

As the signal line 69, a well-known material can be used, and for example, the signal line 69 can be formed of a conductive fiber. As the conductive fiber, it is possible to use one or more fibers selected from the group consisting of metal fiber, metal-coated fiber, carbon fiber, conductive polymer fiber, conductive polymer-coated fiber, and conductive paste-coated fiber. These may be used alone or in combination of two or more kinds thereof.

The metal material of the metal fiber and the metal-coated fiber is not limited as long as it has conductivity. Examples of the metal material include copper, silver, gold, nickel, tin, lead, zinc, bismuth, antimony, stainless steel, aluminum, silver/silver chloride, and alloys of these. These may be used alone or in combination of two or more kinds thereof. Among these, from the viewpoint of conductivity, silver can be used. In addition, it is preferable that the metal material does not include a metal such as chromium, which imposes a burden on the environment.

The fiber material of the metal-coated fiber, the conductive polymer-coated fiber, or the conductive paste-coated fiber described above is not particularly limited and may be any one of synthetic fiber, semisynthetic fiber, or natural fiber. Among these, polyester, nylon, polyurethane, silk, cotton, and the like are preferably used. These may be used alone or in combination of two or more kinds thereof.

Examples of the above-described carbon fiber include a PAN-based carbon fiber and a pitch-based carbon fiber.

As the conductive polymer material of the conductive polymer fiber and the conductive polymer-coated fiber, for example, polythiophene, polypyrrole, polyaniline, polyacetylene, polyphenylene vinylene, polynaphthalene, a mixture of conductive polymers of derivatives of these and a binder resin, or an aqueous solution of a conductive polymer such as PEDOT-PSS ((3,4-ethylenedioxythiophene)-poly(styrene sulfonate)) is used.

The resin material contained in the conductive paste of the conductive paste-coated fiber is not particularly limited, and preferably has elasticity. For example, the resin material includes one or more materials selected from the group consisting of silicone rubber, urethane rubber, fluororubber, nitrile rubber, acrylic rubber, styrene rubber, chloroprene rubber, and ethylene propylene rubber. These may be used alone or in combination of two or more kinds thereof.

A conductive filler in the conductive paste of the conductive paste-coated fiber described above is not particularly limited, and a well-known conductive material may be used. For example, the conductive filler may include one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.

A metal forming the above-described conductive filler is not particularly limited. For example, the metal may include at least one or two or more among copper, silver, gold, nickel, tin, lead, zinc, bismuth, antimony, silver or silver chloride, and alloys thereof. Among these, from the viewpoint of high conductivity or high availability, silver or copper is preferable.

The above-described signal line 69 may be formed of spun yarn obtained by twisting a plurality of linear conductive fibers. As a result, disconnection of the signal line 69 during deformation can be suppressed.

In the present embodiment, coating of conductive fiber means not only covering of the outer surface of the fiber material. In the case of spun yarn obtained by twisting single fibers, the coating of conductive fiber also means that the voids of the fiber in the spun yarn are impregnated with a metal, a conductive polymer, or a conductive paste such that each of the single fibers configuring the spun yarn is coated.

The tensile elongation at break of the signal line 69 is, for example, 1% or greater and 50% or less and preferably 1.5% or greater and 45%. In a case where the tensile elongation at break thereof is in the above-described numerical ranges, extreme deformation of the protruding portion 60 can be suppressed while breakage during deformation is suppressed.

### <Material of electrode portion 80>

The conductive member 70 constituting the electrode portion 80 is, for example, a paste containing a highly conductive metal. The highly conductive metal includes one or more metals selected from the group consisting of copper, silver, gold, nickel, tin, lead, zinc, bismuth, antimony, and alloys of these. In particular, from the viewpoint of availability and conductivity, silver, silver chloride, or copper is preferable.

In a case where the electrode portion 80 is formed of the paste containing a highly conductive metal, the distal end portion 61 of the protruding portion 60 formed of the rubber-like elastic body is dipped (dip-coated) in a paste-like conductive solution containing the highly conductive metal. In this manner, the electrode portion 80 is formed on the surface of the protruding portion 60.

Further, the conductive member 70 (electrode portion 80) serving as a conductive resin layer may be formed by coating the surface of the protruding portion 60 with the conductive solution containing a conductive filler and a solvent. At this time, in a case where the solvent contains the same material (silicone rubber) as the material of the protruding portion 60, the adhesiveness of the conductive member 70 (conductive resin layer) can be enhanced.

By optionally heating and drying the conductive solution, the conductive silicone rubber is obtained. The conductive silicone rubber may be configured such that it does not contain silicone oil. As a result, a decrease in conductivity due to bleeding out the silicone oil to the surface of the electrode portion 80 can be suppressed.

Further, the protruding portion 60 itself may contain the conductive member. For example, the entire protruding portion 60 may be provided with conductive silicone rubber, and the protruding portion 60 may function as the electrode portion 80. In this case, the gel-like member 85 is directly attached to the protruding portion 60.

### <Method of Producing Electrode 50 for Brain Wave Measurement>

An example of a method of producing the electrode 50 for brain wave measurement according to the present embodiment may include the following steps.

First, the above-described silicone rubber-based curable composition is molded by being pressurized and heated using a mold to obtain a molded product consisting of the base portion 51 and the protruding portion 60. Subsequently, the signal line 69 passes through the inside of each of the protruding portions 60 of the obtained molded product using a sewing needle. Thereafter, the protruding portion 60 (predetermined range including the distal end portion 61) of the obtained molded product is dip-coated with a paste-like conductive solution, heated, dried, and post-cured. In this manner, the electrode portion 80 consisting of the conductive member 70 can be formed on the surface of the protruding portion 60.

Subsequently, the spherical gel-like members 85 are attached to the distal end portions 61 of all the protruding portions 60 in which the electrode portion 80 is formed.

In a case where the gel-like member 85 is not expected to be exchangeable, for example, a material which is in a gel state by being irradiated with ultraviolet light or the like and being cured into a predetermined shape (here, a spherical shape) can be used.

In a case where the gel-like member 85 is expected to be exchangeable, the gel-like member 85 in a state of being already cured in a gel state is attached. In a case where the gel-like member 85 is deteriorated due to the use of the electrode 50 for brain wave measurement, the deteriorated gel-like member 85 is removed and replaced with a new gel-like member 85.

As described above, the electrode 50 for brain wave measurement can be produced, and the gel-like member 85 can be used in an optimal state.

In the above-described molding step, the above-described silicone rubber-based curable composition may be introduced into a molding space where the signal lines 69 are arranged such that insert molding of pressurizing and heating the composition may be used.

The features of the present embodiment are summarized as follows.
(1) An electrode for biosignal measurement (here, the electrode 50 for brain wave measurement), which comes into contact with a skin (here, the head portion 99) of a subject to acquire a biosignal (here, a brain wave signal), the electrode including: a protruding portion 60 that protrudes from a base portion 51; an electrode portion 80 that is provided with a conductive member 70 at least in a distal end portion of the protruding portion 60; and a gel-like member 85 that is provided on the protruding portion 60 to cover the electrode portion 80 and has an inside containing moisture. In a case where the surface of the electrode portion 80 is coated with the gel-like member 85 (polymer gel) with excellent drying resistance, stable biosignal measurement (brain wave measurement) can be performed by suppressing contact resistance to be low without depending on a dry state of the skin such as the scalp.
(2) The electrode portion 80 is provided to cover the distal end portion 61 of the protruding portion 60 with the conductive member 70.
(3) The protruding portion 60 is provided with a conductive rubber including the conductive member 70 and functions as the electrode portion 80. That is, the protruding portion 60 itself functions as the electrode portion 80.
(4) The protruding portion 60 has a conical shape.
(5) The gel-like member 85 has a spherical shape.
(6) The gel-like member 85 is accommodated in a watertight member before use.
(7) The gel-like member 85 is exchangeably provided.
(8) A biosignal measurement device (here, the brain wave measurement device 1) according to the present embodiment includes the electrode for biosignal measurement (the electrode 50 for brain wave measurement) described above.
(9) A biosignal measurement method (here, a brain wave detection method) according to the present embodiment includes measuring a biosignal (here, a brain wave) by mounting the biosignal measurement device (here, the brain wave measurement device 1) described above on a subject.

Thus, the embodiments of the present invention have been described above; however, these are only examples of the present invention, and various configurations other than the above-described ones can be employed.

This application claims priority on the basis of Patent Application No. 2022-022658 filed on January 17, 2022, the entire disclosure of which is incorporated herein by reference.

### REFERENCE SIGNS LIST

1 brain wave measurement device (biosignal measurement device)
10 brain wave electrode unit (electrode unit for biosignal)
11 body portion
12 signal extracting unit
13 threaded portion
14 electrode fixing unit
20 frame
21 electrode unit attaching unit
50 electrode for brain wave measurement (electrode for biosignal measurement)
51 base portion
52 protruding portion forming surface
60 protruding portion
61 distal end portion
69 signal line
70 conductive member
80 electrode portion
85 gel-like member

## Claims

1. An electrode for biosignal measurement, which comes into contact with a skin of a subject to acquire a biosignal, the electrode comprising:
a protruding portion that protrudes from a base portion;
an electrode portion that is provided with a conductive member at least in a distal end portion of the protruding portion; and
a gel-like member that is provided on the protruding portion to cover the electrode portion and has an inside containing moisture.

2. The electrode for biosignal measurement according to Claim 1,
wherein the electrode portion is provided to cover the distal end portion of the protruding portion with the conductive member.

3. The electrode for biosignal measurement according to Claim 1,
wherein the protruding portion is provided with a conductive rubber including the conductive member and functions as the electrode portion.

4. The electrode for biosignal measurement according to any one of Claims 1 to 3,
wherein the protruding portion has a conical shape.

5. The electrode for biosignal measurement according to any one of Claims 1 to 4,
wherein the gel-like member has a spherical shape.

6. The electrode for biosignal measurement according to any one of Claims 1 to 5,
wherein the gel-like member is accommodated in a watertight member before use.

7. The electrode for biosignal measurement according to any one of Claims 1 to 6,
wherein the gel-like member is exchangeably provided.

8. The electrode for biosignal measurement according to any one of Claims 1 to 7,
wherein the biosignal is a brain wave.

9. A biosignal measurement device comprising:
the electrode for biosignal measurement according to any one of Claims 1 to 8.

10. A biosignal detection method of measuring a biosignal by mounting the biosignal measurement device according to Claim 9 on a subject.
